# EUROPÄISCHE PATENTANMELDUNG

(11) **EP 1 956 411 A2**
(43) Veröffentlichungstag der Anmeldung: **13.08.2008**
(21) Anmeldenummer: 07405282.0
(22) Anmeldetag: 19.09.2007
(51) Int. Cl.: G02B 27/01

(54) **Elektrooptisches Blendschutzfilter und Blendschutzeinheit für eine tragbare Blendschutzvorrichtung**

(30) Priorität: 19.09.2006 CH 14902006
(71) Anmelder: SPERIAN Welding Protection AG, 9630 Wattwil (CH)
(72) Erfinder: Cottier, Kaspar, CH-8645 Jona (CH)
(74) Vertreter: Frei Patent Attorneys

(57) **Zusammenfassung**

Ein elektrooptisches Blendschutzfilter für beispielsweise eine Schweisserschutzmaske, weist mindestens zwei hintereinander angeordnete Flüssigkristallzellen (20, 30) vom Typ "twisted nematic" (TN) mit Polarisationsschichten (21, 25, 31, 35) auf, wobei die Polarisationsrichtungen von jeweils zwei Polarisationsschichten (21, 25, 31, 35), die einer Flüssigkristallzelle (20, 30) zugeordnet sind, jeweils gegeneinander verdreht sind.

Dabei sind die Polarisationsrichtungen von jeweils zwei Polarisationsschichten(21, 25, 31, 35), die einer Flüssigkristallzelle (20, 30) zugeordnet sind, entkreuzt, das heisst, um einen von 90° verschiedenen Winkel, gegeneinander verdreht sind.

## Beschreibung

Die Erfindung bezieht sich auf das Gebiet der Blendschutzvorrichtungen, wie sie beispielsweise als bei Schweisserschutzmasken oder -Helme Verwendung finden, und insbesondere auf ein elektrooptisches Blendschutzfilter sowie auf eine Blendschutzeinheit für eine tragbare Blendschutzvorrichtung gemäss dem Oberbegriff der entsprechenden unabhängigen Patentansprüche.

### STAND DER TECHNIK

Blendschutzeinheiten für tragbare Blendschutzvorrichtungen wie Schutzmasken und Schutzbrillen sind allgemein bekannt. Moderne Blendschutzvorrichtungen weisen elektrooptische Filter auf, beispielsweise mit einem Flüssigkristallelement, deren Durchlässigkeit automatisch oder manuell angepasst wird. Ein solches Filter soll, neben einer vorgegebenen und optional auch einstellbaren Schutzstufe, auch eine möglichst geringe Blickwinkelabhängigkeit aufweisen.

WO 2004/102265 hat das Ziel, eine symmetrische Blickwinkelabhängigkeit bezüglich der Normalen zum LCD-Element eines Blendschutzfilters zu erreichen. Die Blickwinkelabhängigkeit wird auf die Doppelbrechung in der LCD-Zelle zurückgeführt. Durch Einsatz einer negativ-doppelbrechenden Kompensationsschicht wird diese ausgeglichen. Für eine optimale Wirkung müssen die Parameter der Zelle und der Kompensationsschicht aufeinander abgestimmt sein, und muss die Zelle eine HT (higly twisted) Zelle sein und möglichst nur einen einzigen Modus des Lichts durchlassen.

US 5'515'186 beschreibt eine Schweissermaske mit Anordnungen von Polarisatoren, welche derart aufeinander abgestimmt sind, dass ein Bereich, der in einer Kombination von zwei Polarisatoren heller erscheint, in Kombination mit einem weiteren Polarisator wieder verdunkelt erscheint. Die Flüssigkristallzellen sind vom Typ her ausdrücklich Zellen, die auf Basis schaltbarer Doppelbrechung funktionieren, im Gegensatz zu "twisted nematic" oder TN Zellen, in welcher die Polarisation gedreht wird. Gemäss der US 5'515'186 kann ein von 90° verschiedener Polarisatoren-Winkel gewählt werden, um eine verbleibende Doppelbrechung ("residual birefringence") zu kompensieren, damit bei einem Blickwinkel senkrecht zu den Zellen ein maximaler Kontrast erreicht wird.

US 6,327,010 zeigt ein optisches Filter mit einer TN-Flüssigkristallzelle und einer Kompensationsschicht aus anders gearteten Flüssigkristallen. Es liegen im ganzen Filter nur eine oder zwei Polarisationsschichten vor. Die Anordnung ist für den Einsatz in LCD-Displays vorgesehen. Es sollen hohe Werte für Kontrast und/oder Helligkeit und/oder die Blickwinkelunabhängigkeit von Kontrast und/oder Farbe erreicht werden. In einer Ausführungsform wird dies erreicht, indem bei einer Flüssigkristallzelle zwei Polarisatoren vorliegen, welche um 90°±10° gegeneinander verdreht sind, die Winkel zwischen den Polarisatoren und den Reibrichtungen der TN-Zelle bestimmten Bedingungen entspricht, und mindestens eine Kompensationsschicht zur Kompensation von Differenzen im optischen Pfad der Flüssigkristallzelle vorliegt.

WO 95/29428 zeigt ein Blendschutzfilter mit einer Kombination zweier Flüssigkristallzellen, in einer gegeneinander verdrehten Anordnung, wobei die beiden Flüssigkristallzellen jeweils zwischen zwei sich gegenseitig auslöschenden Polarisationsfiltern angeordnet sind. Die Flüssigkristallzellen bestehen dabei aus Low Twisted Nematic (LTN) Zellen, wie sie z.B. aus FR2728358, US4952030 und US4609255 bekannt sind.

WO 97/15255 beschreibt eine einzelne oder zwei Flüssigkristallzellen, deren Polarisatoren jeweils sich gegenseitig auslöschend respektive senkrecht zueinander stehend angeordnet sind. Zur Verringerung der Blickwinkelabhängigkeit ist ein Retarderfilm zwischen zwei der Polarisatoren angeordnet.

WO 99/53367 zeigt eine Konfiguration für eine einzelne Flüssigkristallzelle eines Bildschirms. Die Zelle weist - in dieser Folge - einen ersten Polarisator, einen ersten Kompensator, der wiederum aus zwei Schichten besteht, eine Flüssigkristallschicht, einen zweiten (zweischichtigen) Kompensator und einen zweiten Polarisator auf. Die beiden Polarisatoren sind zueinander nicht gekreuzt, sondern um 2° bis 6° oder 20° aus der gekreuzten Lage verdreht. Der erste und der zweite Kompensator sind ebenfalls aus der gekreuzten Lage verdreht, vorzugsweise um denselben Winkel wie die beiden Polarisatoren.

### DARSTELLUNG DER ERFINDUNG

Es ist deshalb Aufgabe der Erfindung, ein elektrooptisches Blendschutzfilter und eine Blendschutzeinheit für eine tragbare Blendschutzvorrichtung der eingangs genannten Art zu schaffen, welche eine geringe Blickwinkelabhängigkeit und einen einfachen Aufbau aufweist. Diese Aufgabe lösen ein elektrooptisches Blendschutzfilter und eine Blendschutzeinheit für eine tragbare Blendschutzvorrichtung mit den Merkmalen der entsprechenden unabhängigen Patentansprüche.

Das elektrooptische Blendschutzfilter weist also mindestens zwei (in Richtung der Flächennormalen der Filterebenen) hintereinander angeordnete Flüssigkristallzellen vom Typ "twisted nematic" (TN) mit Polarisationsschichten auf, wobei die Polarisationsrichtungen von jeweils zwei Polarisationsschichten, die jeweils einer Flüssigkristallzelle zugeordnet sind, jeweils nicht parallel sind, das heisst, um mindestens 45° gegeneinander verdreht sind. Das Filter weist ferner eine Ansteuereinrichtung zur elektrischen Ansteuerung der Flüssigkristallzellen auf. Dabei sind die Polarisationsrichtungen von zwei Polarisationsschichten, die jeweils einer Flüssigkristallzelle zugeordnet sind, entkreuzt, das heisst, um einen von 90° verschiedenen Winkel, gegeneinander verdreht.

Mit einer solchen Anordnung ist die Blickwinkelabhängigkeit der Verdunkelung über einen grossen Bereich des Blickwinkels reduziert. Es werden dabei keine zusätzlichen Elemente der Filter benötigt, insbesondere keine zusätzlichen Retarderschichten oder Kompensationsschichten, sondern lediglich dieselben Komponenten wie bei einer herkömmlichen Doppelzelle. Damit resultiert ein einfacher und kostengünstiger Aufbau.

In einer bevorzugten Ausführungsform der Erfindung weist das Blendschutzfilter mindestens zwei Flüssigkristallzellen auf, wobei in genau zwei dieser Flüssigkristallzellen die Polarisationsrichtungen der zwei Polarisationsschichten, die der jeweiligen Flüssigkristallzelle zugeordnet sind, entkreuzt sind.

In einer weiteren bevorzugten Ausführungsform der Erfindung beträgt in der oder den Zellen, welche entkreuzte Polarisatoren aufweisen, der Winkel zwischen
- der Winkelhalbierenden des spitzen Winkels zwischen den Polarisationsrichtungen der entkreuzten Polarisatoren (21, 25; 31, 35) und
- der Winkelhalbierenden der zugeordneten Flüssigkristallschicht (23, 33) weniger als 45°.
Dabei sind die Winkelhalbierenden der zugeordneten Flüssigkristallschicht jeweils gleich der Winkelhalbierenden zwischen den Orientierungen der beiden Orientierungsschichten der Flüssigkristallschicht.

In einer weiteren bevorzugten Ausführungsform der Erfindung sind die beiden Flüssigkristallzellen in ihrem Aufbau zumindest annähernd spiegelsymmetrisch zueinander. Vorzugsweise verwenden sie die gleichen Flüssigkristalle. Damit wird die Symmetrie der Blickwinkelabhängigkeit bezüglich der Flächennormalen verbessert. Die Symmetrieebene liegt in der räumlichen Mitte der beiden Flüssigkristellzellen. Falls die Zellen sich den mittleren Polarisator teilen, liegt die Symmetrieebenein der Mitte dieses Polarisators. Falls die Zellen jeweils eigene, getrennte Polarisatoren in der Mitte des Blendschutzfilters aufweisen, dann liegt die Symmetrieebene in der Mitte zwischen diesen getrennten Polarisatoren.

Der Verdrehwinkel zwischen den Polarisationsrichtungen von jeweils zwei entkreuzten Polarisationsschichten beträgt vorzugsweise 82° bis 89°, insbesondere 85° bis 88°, wobei der spitze Winkel zwischen den Polarisationsrichtungen massgebend ist.

Vorzugsweise fällt, in einer bevorzugten Ausführungsform der Erfindung, die Winkelhalbierende des spitzen Winkels zwischen den Polarisationsrichtungen zweier entkreuzter Polarisatoren mit der Winkelhalbierenden der zugeordneten Flüssigkristallschicht zusammen.

Mit einer Anordnung entsprechend der Erfindung sind Doppelzellen erzeugbar, die eine markant geringere Variation der Schutzstufe (shade number SN) innerhalb eines Sichtkegels mit einem Öffnungswinkel von 60° (das heisst, in jede Richtung um bis zu 30° von der Flächennormalen des Filters abweichend) aufweisen als herkömmliche Filter, die auf TN Zellen basieren.

In einer bevorzugten Ausführungsform der Erfindung weist die Ansteuereinrichtung zur Speisung der Flüssigkristallzellen eine Nichtlinearität zur Kompensation eines nichtlinearen Spannungs-Verdunkelungs-Verhältnisses der Flüssigkristallzellen auf. Die Nichtlinearität ist vorzugsweise in der Steuerwertbestimmung angeordnet, und ist so - im Signalflusspfad - der Nichtlinearität der Zellen vorgeschaltet.

Eine Blendschutzeinheit für eine tragbare Blendschutzvorrichtung weist ein elektrooptisches Blendschutzfilter gemäss der Erfindung auf.

Weitere bevorzugte Ausführungsformen gehen aus den abhängigen Patentansprüchen hervor. Dabei sind Merkmale der Verfahrensansprüche sinngemäss mit den Vorrichtungsansprüchen kombinierbar und umgekehrt.

### KURZE BESCHREIBUNG DER ZEICHNUNGEN

Im folgenden wird der Erfindungsgegenstand anhand von bevorzugten Ausführungsbeispielen, welche in den beiliegenden Zeichnungen dargestellt sind, näher erläutert. Es zeigen jeweils schematisch:
- Figur 1: eine Schutzmaske mit einer Blendschutzkassette;
- Figur 2: eine Schichtabfolge einer Flüssigkristallzelle;
- Figuren 3, 4, 5: Aufsichten auf eine Flüssigkristallzelle zur Veranschaulichung der Orientierungen ihrer Elemente;
- Figuren 6-7: Schichtabfolgen von Doppel-Flüssigkristallzellen;
- Figuren 8- 9: Aufsichten auf Doppel-Flüssigkristallzellen zur Veranschaulichung der Orientierungen ihrer Elemente; und
- Figur 10: einen Verlauf einer Spannungs-Verdunkelungsabhängigkeit.

Die in den Zeichnungen verwendeten Bezugszeichen und deren Bedeutung sind in der Bezugszeichenliste zusammengefasst aufgelistet. Grundsätzlich sind in den Figuren gleiche Teile mit gleichen Bezugszeichen versehen.

### WEGE ZUR AUSFÜHRUNG DER ERFINDUNG

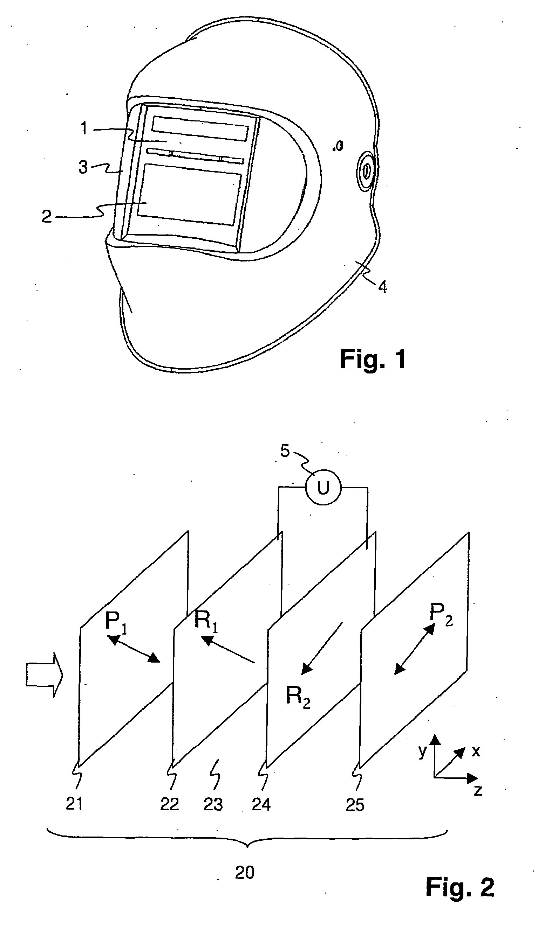

Figur 1 zeigt eine Schweisserschutzmaske 4 mit einer Blendschutzkassette 1. Die Blendschutzkassette 1 ist austauschbar hinter einer ebenfalls auswechselbaren Schutzscheibe 3 angeordnet. Die Blendschutzkassette 1 weist ein elektrooptisches Filter 2 auf, auch Shutter genannt. Das Filter 2 wird durch eine Ansteuerelektronik 5 (in Figur 1 nicht sichtbar) angesteuert, welche Ansteuerelektronik 5 vorzugsweise durch eine Batterie und/oder eine Solarzelle gespeist wird. Vorzugsweise geschieht die Ansteuerung nach Massgabe einer Lichtmenge, die von Sensoren an der Frontseite der Blendschutzkassette 1 erfasst wird. Dabei wird vorzugsweise eine Flackerschaltung zum Detektieren des Lichtbogens eines Schweissvorgängen eingesetzt, so dass beim Auftreten eines Schweissvorgangs das Filter 2 verdunkelt wird. Die Verdunkelung kann nur ein- und ausgeschaltet werden, es kann aber auch die Stärke der Verdunkelung geregelt werden, beispielsweise nach Massgabe der Umgebungshelligkeit, der Intensität des Lichtbogens, einer Benutzervorgabe, etc.

Figur 2 zeigt eine Schichtabfolge einer Flüssigkristallzelle 20. Licht, durch einen Blockpfeil angedeutet, fällt in Richtung einer z-Achse in die Zelle. Die verschiedenen Schichten der Zelle erstrecken sich jeweils in Ebenen parallel zu x- und y-Richtungen. Die Flüssigkristallzelle 20 weist, in Richtung des einfallenden Lichtes gesehen, einen ersten Polarisator 21 auf, gefolgt von einem Substrat mit einer ersten Orientierungsschicht 22 und einem Substrat mit einer zweiten Orientierungsschicht 24. Die Orientierungsschichten dienen in bekannter Weise zur Orientierung der Flüssigkristallmoleküle an der Grenze zum jeweiligen Substrat. Zwischen den Orientierungsschichten 22, 24 liegt ein Zwischenraum mit einer Flüssigkristallschicht 23. Anschliessend an die zweite Orientierungsschicht 24 ist ein zweiter Polarisator 25 angeordnet. Die beiden Substrate 22, 24 sind als Elektroden zur Ansteuerung der Flüssigkristallschicht 23 ausgebildet und sind elektrisch mit der Ansteuerelektronik 5 verbunden und durch diese ansteuerbar.

Die charakteristischen Orientierungen der Polarisatoren und Orientierungsschichten sind durch Pfeile angedeutet. Die Richtungen der ersten und der zweiten Orientierungsschicht 22, 24 sind mit R1 und R2 bezeichnet. Die Substrate wiederum sind zwischen zwei Polarisatoren angebracht. Die Polarisationsrichtungen der ersten und der zweiten Polarisatioinsschichten 21, 25 sind entlang der Richtungen P1 und P2 orientiert

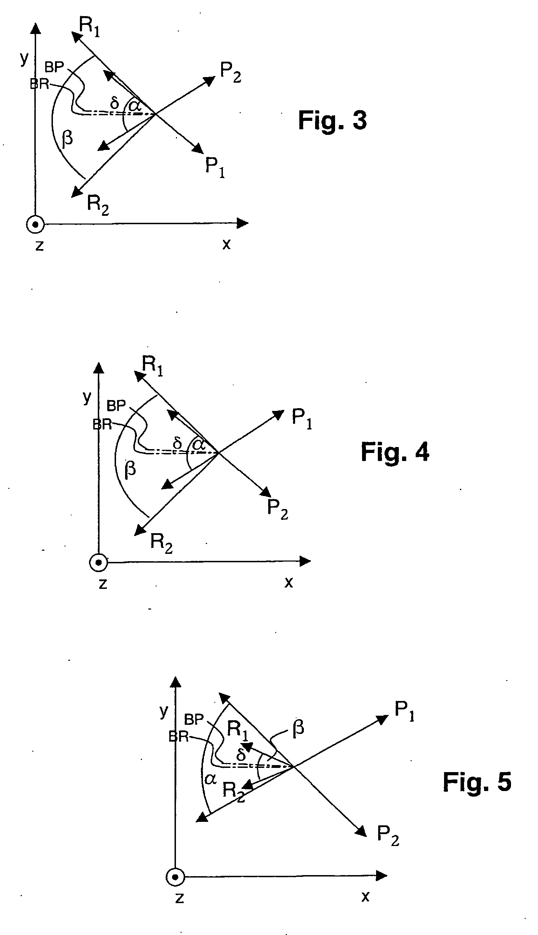

Figur 3, 4 und 5 zeigen die Richtungen der Polarisatoren und Orientierungsschichten einer Flüssigkristallzelle in der Projektion auf die x/y Ebene. Die gegenseitige Verdrehung der Polarisationsrichtungen P1,P2 ist mit *α* bezeichnet. Dabei ist der Winkel definiert als der kleinere der beiden möglichen Verdrehungswinkel, und kann somit maximal 90° betragen. Die gegenseitige Verdrehung der Orientierungsrichtungen R1, R2 ist mit β bezeichnet, und wird auch "twist" der Flüssigkristallzelle genannt.

In einem herkömmlichen Schweisserschutzfilter beträgt der Winkel zwischen den Polarisationsrichtungen α = 90°. Diese werden gewöhnlich als gekreuzte (crossed) oder gegenseitig auslöschende (mutually extinguishing) Polarisatoren bezeichnet. Mit dieser Anordnung wird eine maximale Auslöschung erreicht, wenn sich die Flüssigkristallzelle im aktivierten Zustand befindet, also die Moleküle nahezu senkrecht angeordnet sind und das Licht die Flüssigkristallzelle ohne wesentliche Änderung seiner Polarisation durchquert.

Durch eine gezielte Entkreuzung der Polarisatoren, so dass beispielsweise 82° < α < 89°, ergibt sich eine veränderte Blickwinkelabhängigkeit der einzelnen Zelle. Dabei wird in der Richtung, die gewöhnlich als "best viewing direction" bezeichnet wird, die Verdunkelung verstärkt, typischerweise in einem Winkelbereich zwischen 5° und 45° von der Flächennormalen entfernt. Kombiniert man eine derart ausgestaltete Zelle mit einer zweiten Flüssigkristallzelle entsprechend der Erfindung, gleicht die verstärkte Abdunkelung der ersten Zelle die Zonen mit abgeschwächter Verdunkelung der zweiten Zelle aus, und umgekehrt. Im gesamten wird also die Abdunkelung - über einen bestimmten Blickwinkelbereich gemessen - besser ausgeglichen als bei einer herkömmlichen Konfiguration der Doppelzelle mit gekreuzten Polarisatoren. Der maximal erreichbare Kontrast wird zwar mit dem Entkreuzen reduziert, dies ist aber insofern nicht störend, da die übliche maximale Schutzstufe von 13 eines Schweisserschutzfilters ohne weiteres erreicht werden kann. Im Gegenteil ergibt sich sogar ein weiterer Vorteil aus diesem abgeschwächten maximalen Kontrast, da dadurch störende Bereiche mit zu starker Abdunkelung wegfallen.

Figur 3 zeigt die Konfiguration einer bevorzugten Ausführung einer Flüssigkristallzelle gemäss der Erfindung, basierend auf einer TN-Zelle mit β=90°, bei welcher die Orientierungen der Polarisatoren zumindest annähernd parallel zu jenen der jeweils benachbarten Orientierungsschichten verlaufen (in der Folge "P-buffed" genannt). Die Winkelhalbierende des Twistwinkels β ist mit BR bezeichnet, die Winkelhalbierende der Verdrehung der Polarisatoren α als BP. Der Winkel zwischen den beiden Winkelhalbierenden ist mit δ bezeichnet. In der bevorzugten Ausführung der Erfindung ist der Winkel zwischen den Winkelhalbierenden klein, zum Beispiel δ<45°, oder sind die Winkelhalbierenden sogar zumindest annähernd parallel, zum Beispiel im Bereich 0°<δ<20°.

Eine weitere bevorzugte Ausführungsform zeigt Figur 4, bei welcher die "X-buffed" Konfiguration gewählt wurde, d.h. bei der die Orientierungen der Polarisatoren zumindest annähernd senkrecht zu jenen der jeweils benachbarten Orientierungsschichten stehen.

Figur 5 zeigt eine weitere Ausführung in der X-buffed Konfiguration, bei der eine low twisted nematic Zelle, mit β<85°, zum Einsatz kommt.

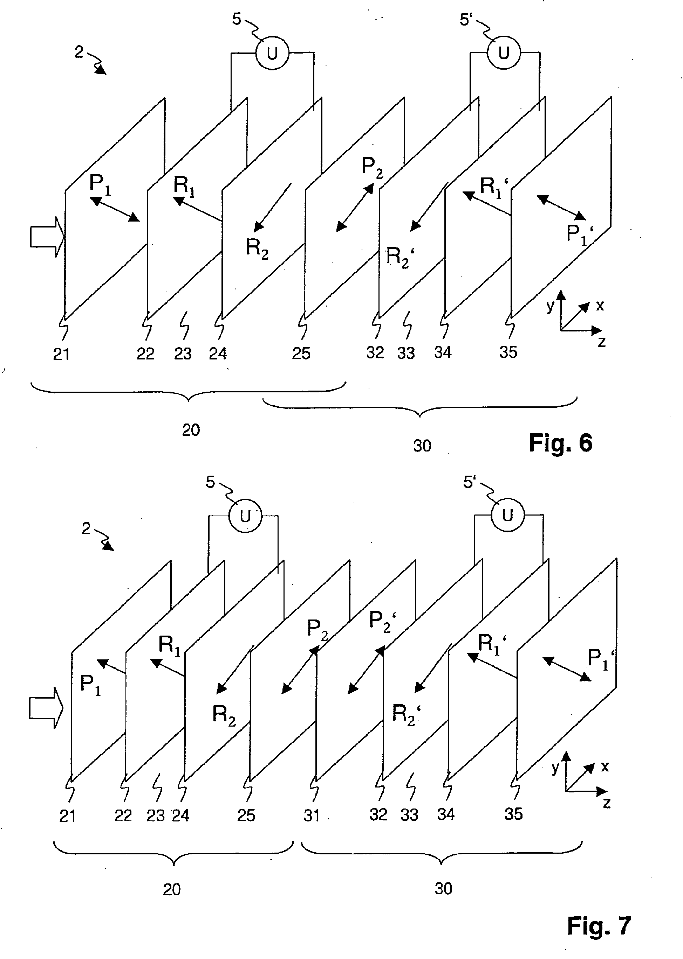

Figur 6 zeigt eine Schichtabfolge einer Doppel-Flüssigkristallzelle, wobei sowohl die erste Flüssigkristallzelle 20 als auch eine zweite Flüssigkristallzelle 30 entkreuzt sind. Die zweite Flüssigkristallzelle 30 teilt sich den zweiten Polarisator 25 mit der ersten Flüssigkristallzelle 20 und weist dazu weitere Substrate mit einer dritten Orientierungsschicht 32 und mit einer vierten Orientierungsschicht 34 auf, zwischen welchen ein zweiter Zwischenraum mit einer zweiter Flüssigkristallschicht 33 liegt. Ein dritter Polarisator 35 ist anschliessend an die vierte Orientierungsschicht 34 angeordnet. Die beiden Substrate 32, 34 sind als Elektroden zur Ansteuerung der zweiten Flüssigkristallschicht 33 ausgebildet und sind elektrisch mit der Ansteuerelektronik 5 oder mit einer weiteren Ansteuerelektronik 5' verbunden und durch diese ansteuerbar.

Bei der in Figur 6 gezeigten P-buffed-Zelle sind zudem P1 und R1 im wesentlichen parallel, sowie auch P2 und R2. In einer X-buffed-Zelle (nicht illustriert) würden P1 und R1, sowie P2 und R2 jeweils im wesentlichen senkrecht aufeinuander stehen..

Die Anordnung der beiden Zellen ist also spiegelsymmetrisch zueinander, und die beiden Flüssigkristallzellen sind mit Flüssigkristallen unterschiedlicher Drehrichtung (chirality) gefüllt. Die Symmetrieebene befindet sich in der Mitte zwischen den beiden Zellen respektive des gemeinsamen Polarisators 25.

Figur 7 zeigt eine Schichtabfolge einer Doppel-Flüssigkristallzelle in einer anderen bevorzugten Ausführungsform der Erfindung. Bei dieser ist der zweite Polarisator 25 nicht beiden Zellen 20, 30 gemeinsam, sondern die zweite Flüssigkristallzelle 30 weist einen weiteren mittleren (oder einen vierten) Polarisator 31 auf, der zwischen dem zweiten Polarisator 25 und der dritten Orientierungsschicht 32 angeordnet ist. Der vierte Polarisator 31 weist eine Orientierung P2' auf, welche im wesentlichen parallel zur Orientierung P2 ist. Die beiden Zellen 20, 30 können also separat voneinander gefertigt und geprüft werden und erst anschliessend zu einer Doppelzelle kombiniert werden.

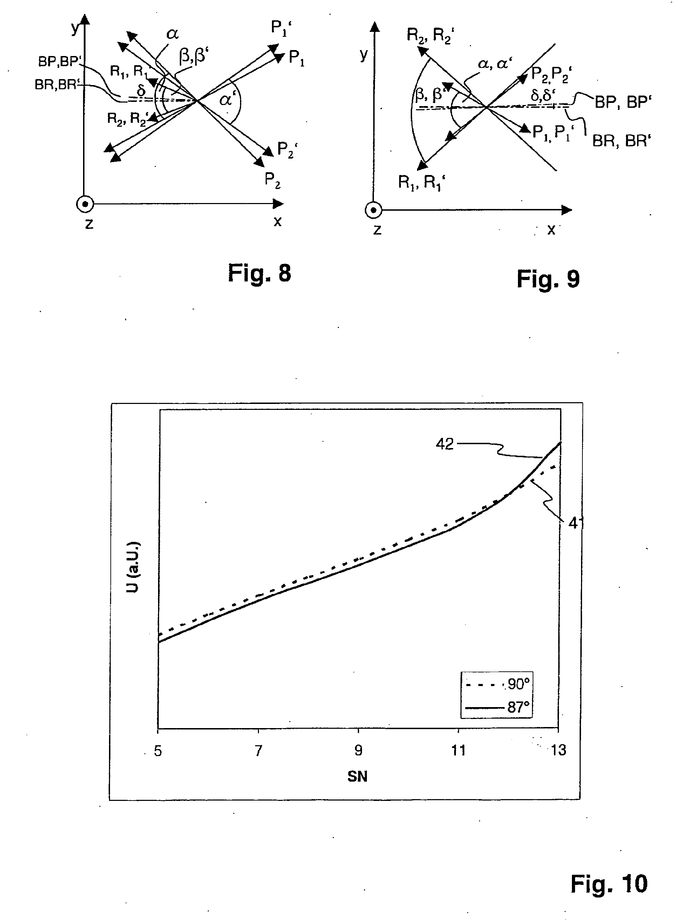

Figur 8 zeigt die verschiedenen Richtungen und Orientierungen innerhalb der Doppelzelle gemäss den beiden letztgenannten bevorzugten Ausführungsformen der Erfindung. Im Gegensatz zur Figur 7 ist in Figur 8 eine X-buffed, LTN Anordnung dargestellt.Dabei sind die Richtungen der Orientierungsschichten der Substrate 32, 34 und eines dritten Polarisators 35 entsprechend den Richtungen der ersten Flüssigkristallzelle 20 folgendermassen ausgerichtet:
- Die Orientierung R2' der dritten Orientierungsschicht 32 zumindest annähernd gleichgerichtet zur Orientierung R2 der zweiten Orientierungsschicht 24.
- Die Orientierung R1' der vierten Orientierungsschicht 34 zumindest annähernd gleichgerichtet zur Orientierung R1 der ersten Orientierungsschicht 22.
- Die Orientierung P1' des dritten Polarisators 35 zumindest annähernd parallel zur Orientierung P1 des ersten Polarisators 21.
- Die Orientierung P2' des vierten Polarisators 31 zumindest annähernd parallel zur Orientierung P2 des zweiten Polarisators 25.
- Die Orientierung P2 des zweiten Polarisators 25 gegenüber der Richtung P1 des ersten Polarisators 21 entkreuzt mit Winkel *α*<90°, insbesondere 82° < *α* < 89°. P1 steht also nicht senkrecht auf P2
- Für die dargestellte Ausführung mit vier Polarisatoren: Die Orientierung P1' des dritten Polarisators 35 ist gegenüber der Richtung P2' des vierten Polarisators 31 ebenfalls entkreuzt, P1' steht also nicht senkrecht auf P2'.

Dabei sind die jeweiligen Richtungspaare, welche als "zumindest annähernd parallel" bezeichnet wurden, wie zum Beispiel P1 und P1', bewusst als leicht gegeneinander verdreht dargestellt. Die gegenseitige Verdrehung ist zum Beispiel kleiner als +/- 20°. Dies gilt sowohl für die gegenseitige Verdrehung der Orientierungsschichten als auch für die gegenseitige Verdrehung der Polarisatoren.

Für die Ausführung mit drei Polarisatoren (nicht dargestellt) würde sinngemäss gelten, dass die Orientierung P1' des dritten Polarisators 35 gegenüber der Richtung P2 des zweiten Polarisators 25 entkreuzt ist, P1' steht also in diesem Fall nicht senkrecht auf P2.

Figur 9 zeigt eine bevorzugte Ausführung aufbauend auf zwei X-buffed TN Zellen, in welcher im Rahmen der entsprechenden Produktionstoleranzen gilt:
- Die Orientierung R2' der dritten Orientierungsschicht 32 ist gleichgerichtet zur Orientierung R2 der zweiten Orientierungsschicht 24.
- Die Orientierung R1' der vierten Orientierungsschicht 34 ist gleichgerichtet zur Orientierung R1 der ersten Orientierungsschicht 22.
- Die Orientierung P1' des dritten Polarisators 35 ist parallel zur Orientierung P1des ersten Polarisators 21.
- Die Orientierung P2' des vierten Polarisators 25 ist parallel zur Orientierung P2 des zweiten Polarisators 31.
- Die Orientierung P2 des zweiten Polarisators 25 ist gegenüber der Richtung P1 des ersten Polarisators 21 entkreuzt mit *α*<90°, insbesondere 82° < *α* < 89°.
- Für die dargestellte Ausführung mit vier Polarisatoren: Die Orientierung P1' des dritten Polarisators 35 ist gegenüber der Richtung P2' des vierten Polarisators 31 entkreuzt mit *α*<90°, insbesondere 82° < *α* < 89°. P1' steht also nicht senkrecht auf P2'.

Für die Ausführung mit drei Polarisatoren, bei welcher der zweite Polarisator 25 mit dem vierten Polarisator 31 identisch ist (nicht dargestellt) würde sinngemäss gelten, dass die Orientierung P1' des dritten Polarisators 35 gegenüber der Richtung P2 des zweiten Polarisators 25 entkreuzt ist, P1' steht also in diesem Fall nicht senkrecht auf P2.

Die in der Figur 8 dargestellte Konfiguration benutzt zwei Zellen vom Typ X-buffed LTN, die in der Figur 9 dargestellte Konfiguration benutzt zwei Zellen vom Typ X-buffed TN. Die obigen Angaben sind jeweils auch für P-buffed und TN bzw. LTN Typen gültig, um eine Doppelzelle gemäss der Erfindung herzustellen.

Um aus einem herkömmlichen für Schweisserschutzfilter verwendeten Filter einen Lichtshutter gemäss den dargestellten bevorzugten Ausführungsformen der Erfindung herzustellen, müssen also die zwei zu einer Flüssigkristallzelle gehörigen Polarisatoren gegeneinander entkreuzt werden. Dies kann zum Beispiel erreicht werden, indem
- die äusseren Polarisatoren 21, 35 je in Richtung der Winkelhalbierenden BR, BR' um 90°-*α* zueinander verdreht werden,
- oder aber, indem der eine mittlere Polarisator oder die beiden mittleren Polarisatoren in Richtung der Winkelhalbierenden BR, BR' um 90°-*α* verdreht werden,
- oder indem alle (d.h.drei oder vier) Polarisatoren bezüglich der üblichen Orientierung verdreht werden, und zwar so dass der resultierende Winkel zwischen den Polarisationsrichtungen von jedem der äusseren Polarisatoren 21, 35 und dem einen mittleren Polarisator 25 - respektive dem jeweils zugeordneten von zwei mittleren Polarisatoren 25, 31- jeweils *α*<90° beträgt, wobei insbesondere 82° < *α* < 89°, und wobei ferner jeweils die Winkelhalbierenden BR und BP sowie die Winkelhalbierenden BR' und BP' nur wenig gegeneinander verdreht sind, also 0°< δ, δ'<20°.

Dadurch ergibt sich eine besonders gute Kompensation der erwähnten verstärkten dunklen Bereiche der einen Zelle mit den hellen Bereichen der zweiten, und umgekehrt. Mit der speziellen Bedingung innerhalb des einzelnen Flüssigkristallzellen, nämlich dass δ,δ' relativ klein sind, zum Beispiel δ,δ' <45°, wird erreicht dass die Winkelbereiche der "best viewing direction" verstärkt verdunkelt werden. Mit der speziellen Bedingung zur Anordnung der beiden Flüssigkristallzellen, nämlich dass die Zellen im wesentlichen punktsymmetrisch angeordnet sind, wird erreicht, dass die helleren Zonen der einen Zelle mit den so so enstandenen verstärkten Abdunkelungen der anderen Zelle kompensiert werden.

In einer weiteren bevorzugten Ausführungsform der Erfindung ist nur eine der beiden Zellen einer Doppelzelle entkreuzt. In dieser Ausführung wird eine asymmetrische Blickwinkelabhängigkeit erreicht, was im Augeschutz interessant sein kann, da das vertikale Sichtfeld der Augen nach oben signifikant kleiner ist als nach unten. In dieser Ausführungsform ist also vorzugsweise die eine Zelle dermassen ausgerichtet, dass die "best viewing direction" nach unten zeigt. Diese Zelle ist mit entkreuzten Polarisatoren ausgestaltet. Die andere Zelle ist eine Standardzelle mit gekreuzten Polarisatoren, deren "best viewing direction" nach oben zeigt. Dabei zeigt die "best viewing direction" einer Zelle ungefähr in Richtung der Winkelhalbierenden BR der zugeordneten Orientierungsschichten.

Die Zellen der bisher vorgestellten Ausführungsformen sind vorzugsweise vom Typ Twisted Nematic (TN) mit einem Twist, also mit einem Winkel β zwischen den Orientierungsschichten, zwischen 70°<β<110°, wobei die Richtung des Twists in der einen Zelle der Richtung in der anderen Zelle entgegengesetzt ist.

In einer weiteren bevorzugten Konfiguration handelt es sich bei der oder den entkreuzten Zellen der bisher vorgestellten Ausführungsformen um "low-twisted nematic" (LTN) Zellen mit einem Twist von rund 80° und vorzugsweise zwischen 70°<β<85°.

In einer bevorzugten Konfiguration handelt es sich bei der oder den entkreuzten Zellen um sogenannte X-Buffed Zellen, d.h. Zellen in welchen die Orientierungsschichten jeweils um zumindest annähernd 90° gegenüber den Polarisationsrichtungen der jeweils nächstliegenden Polarisatoren verdreht sind.

In einer bevorzugten Konfiguration besteht das Filter 2 aus zwei symmetrischen Zellen 20, 30, die beide vom entkreuzten Typ sind. Sie werden vorzugsweise beide mit derselben Spannung und durch dieselbe Ansteuerelektronik 5 angesteuert. Die mindestens zwei Flüssigkristallzellen sind also mit derselben Versorgungsspannungsquelle verbunden.

Figur 10 zeigt einen Verlauf einer Spannungs-Verdunkelungsabhängigkeit. Dabei ist entlang der Abszisse die Verdunkelung SN ("shade number") durch ein Filter in Abhängigkeit der Amplitude U der Ansteuerspannung aufgetragen. Für gekreuzte Polarisatoren, also mit *α* = 90° ergibt sich ein erster Kurvenverlauf 41. Für ungekreuzte Polarisatoren, im vorliegenden beispielhaften Fall mit *α* = 87° ergibt sich ein zweiter Kurvenverlauf 42, welcher wesentlich stärker von einer idealen linearen Beziehung abweicht.

Der Verlauf ist typisch für eine spiegelsymmetrisch ausgebildete und angeordnete Doppel-TN-Zelle in der X-buffed Ausführung mit vier Polarisatoren, bei welcher im Rahmen der Fertigungstoleranzen die Polarisationsrichtungen P1 mit P2 und P1' mit P2' sowie die Reibrichtungen R1 mit R1' und R2 mit R2' zusammenfallen. Je nach Ausführungsform, gewünschten Schutzstufen und gewählten Komponenten fällt die Nichtlinearität mehr oder weniger ausgeprägt aus, sie kann in einigen Fällen auch entfallen (zum Beispiel wenn der Filter von Figur 10 nur bis zur Schutzstufe 11 betrieben wird).

Die Zellen werden im allgemeinen mit einer um den Nullpunkt symmetrischen Rechteckspannung angesteuert.. Typische Frequenzen des Rechtecksignals sind 40-50 Hz, können aber bei energiesparenden Filtern auch bis zu und weniger als 1 Hz betragen.

Bevorzugt werden insgesamt spiegelsymmetrisch angeordnete LTN Flüssigkristallzellen in der X-buffed Konfiguration angeordnet, mit insgesamt vier Polarisatoren, bei denen der charakteristische Winkel δ mindestens annähernd null ist und bei welcher die Polarisationsrichtungen P1 mit P2 und P1' mit P2' sowie die Reibrichtungen R1 mit R1' und R2 mit R2' zusammenfallen.

Um trotz des allfälligen nichtlinearen Spannungs-Verdunkelungsverhaltens ein lineares Gesamtverhalten bezüglich einer Benutzervorgabe oder eines Steuersignals einer Regelung zu erhalten, weist die Ansteuerung zur Kompensation eine Nichtlinearität auf. Im einfachsten Fall ist dies beispielsweise eine nichtlineare Skalierung der Stellung eines vom Benutzer eingestellten Eingabemittels, beispielsweise eines Drehknopfes oder eines Schiebereglers. In einer anderen Ausführungsform der Erfindung wird die Versorgungsspannung durch eine digitale oder analoge Elektronik gebildet, welche eine Kompensationskennlinie gespeichert hat. Die Kompensationskennlinie ist invers zur Spannungs-Verdunkelungs-Kennlinie geformt, so dass die Serieschaltung der Kompensationskennlinie mit der Spannungs-Verdunkelungs- Kennlinie eine linearen Verlauf ergibt.

Die Flüssigkristallzellen können neben den herkömmlichen TN oder STN LC-Mischungen auch guest-host LCs enthalten. Die Zellen können so präpariert sein, damit eine niedrige Verankerungsenergie der Randmoleküle erreicht wird, wie beispielsweise in der Europäischen Patentanmeldung mit der Anmeldenummer 06 405 072 der gleichen Anmelderin beschrieben ist.

Die Zellen können auch mit anderen Komponenten, zum Beispiel mit zusätzlichen mit guest-host LC Mischungen gefüllten Zellen, oder weiteren TN Zellen, kombiniert werden.

### BEZUGSZEICHENLISTE

- 1: Blendschutzkassette
- 2: Filter
- 3: Schutzscheibe
- 4: Schutzmaske
- 5, 5': Ansteuerung, Spannungsquelle
- 20: erste Flüssigkristallzelle
- 21: erster Polarisator
- 22: erste Orientierungsschicht
- 23: Zwischenraum mit Flüssigkristallschicht
- 24: zweite Orientierungsschicht
- 25: zweiter Polarisator
- 30: zweite Flüssigkristallzelle
- 31: vierter Polarisator
- 32: dritte Orientierungsschicht
- 33: Zwischenraum mit Flüssigkristallschicht
- 34: vierte Orientierungsschicht
- 35: dritter Polarisator

## Patentansprüche

1. Elektrooptisches Blendschutzfilter (2), aufweisend
• mindestens zwei hintereinander angeordnete Flüssigkristallzellen (20, 30) vom Typ "twisted nematic" (TN) mit Polarisationsschichten (21, 25, 31, 35),
wobei die Polarisationsrichtungen von jeweils zwei Polarisationsschichten (21, 25, 31, 35), die jeweils einer Flüssigkristallzelle (20, 30) zugeordnet sind, jeweils um mindestens 45° gegeneinander verdreht sind und
• eine Ansteuereinrichtung (5, 5') zur elektrischen Ansteuerung der Flüssigkristallzellen (20, 30),
**dadurch gekennzeichnet, dass**
• die Polarisationsrichtungen von zwei Polarisationsschichten (21, 25, 31, 35), die jeweils einer Flüssigkristallzelle (20, 30) zugeordnet sind, entkreuzt, das heisst, um einen von 90° verschiedenen Winkel, gegeneinander verdreht sind.

2. Elektrooptisches Blendschutzfilter (2) gemäss Anspruch 1, aufweisend mindestens zwei Flüssigkristallzellen, wobei
• in **genau zwei** dieser Flüssigkristallzellen (20, 30) die Polarisationsrichtungen der zwei Polarisationsschichten (21, 25; 31, 35), die der jeweiligen Flüssigkristallzelle (20, 30) zugeordnet sind, entkreuzt sind.

3. Elektrooptisches Blendschutzfilter (2) gemäss Anspruch 1 oder 2, wobei in der oder den Zellen, welche entkreuzte Polarisatoren aufweisen, der **Winkel zwischen**
• der **Winkelhalbierenden** des spitzen Winkels zwischen den Polarisationsrichtungen der entkreuzten Polarisatoren (21, 25; 31, 35) und
• der **Winkelhalbierenden** der zugeordneten Flüssigkristallschicht (23, 33) weniger als 45° beträgt.

4. Elektrooptisches Blendschutzfilter (2) gemäss einem der Ansprüche 2 bis 3, wobei die **beiden Flüssigkristallzellen** (20, (30)) in ihrem Aufbau zumindest annähernd spiegelsymmetrisch zueinander sind, wobei insbesondere gilt, dass das Blendschutzfilter, in dieser Reihenfolge, aufweist:
• einen ersten Polarisator (21),
• ein Substrat mit einer ersten Orientierungsschicht (22),
• einen Zwischenraum mit Flüssigkristallschicht (23),
• ein Substrat mit einer zweiten Orientierungsschicht (24),
• einen zweiten Polarisator (25),
• einen vierten Polarisator (31), der mit dem zweiten Polarisator (25) identisch sein kann,
• ein Substrat mit einer dritten Orientierungsschicht (32),
• einen weiteren Zwischenraum mit Flüssigkristallschicht (33),
• ein Substrat mit einer vierten Orientierungsschicht (34),
• einen dritten Polarisator (35),
wobei
• die Orientierung R2' der dritten Orientierungsschicht (32) zumindest annähernd gleichgerichtet zur Orientierung R2 der zweiten Orientierungsschicht (24) ist;
• die Orientierung R1' der vierten Orientierungsschicht (34) zumindest annähernd gleichgerichtet zur Orientierung R1 der ersten Orientierungsschicht (22) ist;
• die Orientierung P1' des dritten Polarisators (35) zumindest annähernd parallel zur Orientierung P1 des ersten Polarisators (21) ist;
• die Orientierung P2' des vierten Polarisators (31) zumindest annähernd parallel zur Orientierung P2 des zweiten Polarisators (25) ist;
• die Orientierung P2 des zweiten Polarisators (25) gegenüber der Richtung P1 des ersten Polarisators (21) entkreuzt mit Winkel α<90° ist, insbesondere mit 82° < *α* < 89°;
• die Orientierung P1' des dritten Polarisators (35) gegenüber der Richtung P2' des vierten Polarisators (31) entkreuzt mit Winkel α<90° ist, insbesondere mit 82° *< α* < 89°.

5. Elektrooptisches Blendschutzfilter (2) gemäss Anspruch 4, wobei
• die Orientierung R2' der dritten Orientierungsschicht (32) gleichgerichtet zur Orientierung R2 der zweiten Orientierungsschicht (24) ist;
• die Orientierung R1' der vierten Orientierungsschicht (34) gleichgerichtet zur Orientierung R1 der ersten Orientierungsschicht (22) ist;
• die Orientierung P1' des dritten Polarisators (35) parallel zur Orientierung P1 des ersten Polarisators (21) ist;
• die Orientierung P2' des vierten Polarisators (31) parallel zur Orientierung P2 des zweiten Polarisators (25) ist.

6. Elektrooptisches Blendschutzfilter (2) gemäss einem der vorherigen Ansprüche, wobei der Verdrehwinkel zwischen den Polarisationsrichtungen von jeweils zwei entkreuzten Polarisationsschichten (21, 25; 31, 35) **82° bis 89°,** vorzugsweise **85° bis 88°**, beträgt, wobei der spitze Winkel zwischen den Polarisationsrichtungen massgebend ist.

7. Elektrooptisches Blendschutzfilter (2) gemäss einem der vorherigen Ansprüche, wobei die Flüssigkristallzellen (20, 30) "**X-buffed**"-Zellen sind, das heisst, dass die Orientierungsschichten jeweils um zwischen 70° und 90° gegenüber den Polarisationsrichtungen der jeweils nächstliegenden Polarisatoren (21, 25, 31, 35) verdreht sind.

8. Elektrooptisches Blendschutzfilter (2) gemäss einem der vorhergehenden Ansprüche, wobei die **Winkelhalbierende** des spitzen Winkels zwischen den Polarisationsrichtungen zweier entkreuzter Polarisatoren (21, 25; 31, 35) mit der Winkelhalbierenden der zugeordneten Flüssigkristallschicht (23, 33) **zusammenfällt.**

9. Elektrooptisches Blendschutzfilter (2) gemäss einem der vorherigen Ansprüche, welches **keine Retarderschichten** oder Kompensationsschichten zur Kompensation einer Doppelbrechung in mindestens einer Flüssigkristallschicht (23, 33) aufweist.

10. Elektrooptisches Blendschutzfilter (2) gemäss einem der vorherigen Ansprüche, wobei die Flüssigkristallzellen (20, 30) vom Typ "**twisted nematic**" (TN) mit einem Verdrehwinkel (Twist) zwischen 70° und 110° sind, und der Verdrehwinkel in der einen Zelle (20) dem Verdrehwinkel in der anderen Zelle (30) entgegengesetzt ist.

11. Elektrooptisches Blendschutzfilter (2) gemäss Anspruch 7, wobei eine oder beide der Flüssigkristallzellen (20, 30) vom Typ "**low-twisted nematic**" (LTN) sind, mit einem Verdrehwinkel (Twist) zwischen 10° und 85°, und vorzugsweise zwischen 70° und 85°.

12. Verfahren zum Betrieb eines elektrooptischen Blendschutzfilters (2) gemäss einem der vorherigen Ansprüche, wobei die mindestens zwei Flüssigkristallzellen (20, 30) mit **derselben Ansteuerspannung** angesteuert werden.

13. Verfahren zum Betrieb eines elektrooptischen Blendschutzfiltesr (2) gemäss einem der Ansprüche 1 bis 11, wobei eine Nichtlinearität im Spannungs-Verdunkelungs-Verhältnis mindestens einer der Flüssigkristallzellen (20, 30) durch eine **Nichtlinearität** der Ansteuereinrichtung (5) kompensiert wird.

14. Blendschutzeinheit (1) für eine tragbare Blendschutzvorrichtung (4), **dadurch gekennzeichnet, dass** die Blendschutzeinheit (1) ein elektrooptisches Blendschutzfilter (2) nach einem der Ansprüche 1 bis 11 aufweist.
